# EUROPEAN PATENT SPECIFICATION

(11) **EP 3 056 238 B1**
(45) Date of publication and mention of the grant of the patent: **28.08.2019**
(21) Application number: 16153562.0
(22) Date of filing: 01.02.2016
(51) Int. Cl.: A61M 25/00, A61M 25/01

(54) **CATHETER**
KATHETER
CATHÉTER

(30) Priority: 13.02.2015 JP 2015026948; 11.12.2015 JP 2015242283
(43) Date of publication of application: 17.08.2016
(73) Proprietor: Asahi Intecc Co., Ltd., Seto-shi, Aichi 489-0071 (JP)
(72) Inventor: MATSUMOTO, Satoru, Seto-shi, Aichi 489-0071 (JP); HORI, Takayuki, Seto-shi, Aichi 489-0071 (JP)
(74) Representative: Winter, Brandl, Fürniss, Hübner, Röss, Kaiser, Polte - Partnerschaft mbB

(56) References cited:
- EP-A2- 0 033 659
- WO-A1-01/62331
- WO-A1-2013/179103
- JP-A- 2009 268 648
- JP-A- 2012 196 275
- US-A- 5 253 653
- US-A- 5 810 837
- US-A1- 2003 125 711
- US-A1- 2006 201 601

## Description

### Field

The present invention relates to a catheter for medical use.

### Background

Conventionally, there is known a catheter with a radiopaque marker provided at a distal end of the catheter so that the distal end position of the catheter can be recognized visually during procedures.

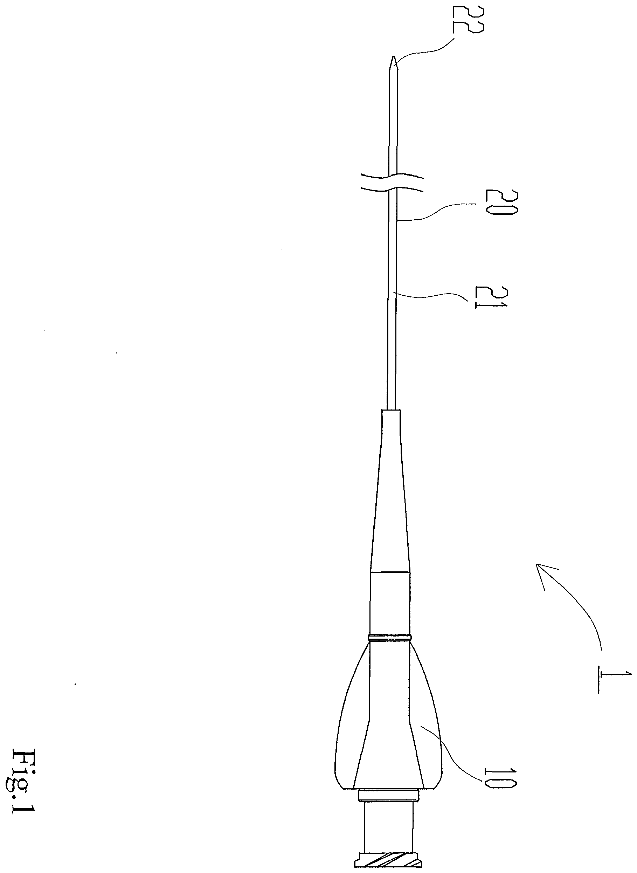

For example, JP(S)64-68276 A discloses a catheter including an elongate main tubular body 10 with a polytetrafluoroethylene core 16 and a polyurethane outer jacket 18 covering the polytetrafluoroethylene core 16, and a soft pointed tip 12 adhered to a distal end of the main tubular body 10, in which at a portion where the main tubular body 10 and the soft pointed tip 12 are connected to each other, a radiopaque precious metal marker band 14 is provided (see the upper right column in page 5, FIG. 1, etc.).

Moreover, JP 2006-34347 A discloses a catheter tube for medical use including a resin inner layer tube, a reinforcing material layer wound around the periphery of the inner layer tube, and an outer layer tube covering the inner layer tube and the reinforcing material layer, in which an X-ray impermeable marker is arranged to be in contact with the reinforcing material layer (see FIG. 20, etc.).

Further, US 5 810 837 A discloses an endoprosthesis inserted under tension in a recess of a flexible inner catheter and secured by means of a tube-shaped outer catheter. The recess is formed by a connector piece that connects a tip to a piece of tubing of the inner catheter.

WO 2013 179103 A1 discloses a medical devices with an imaging marker distal to lumen openings.

US 2006 201601 A1 discloses a flexible marker material that is visible to at least one form of electromagnetic wave, sound wave and/or magnetic wave. The marker material is used on a medical device and adds little or no bulk to the medical device.

US 2003 125711 A1 discloses a catheter having a catheter lumen defined by a catheter wall wherein at least a portion of the catheter wall has at least one radiopaque member.

WO 01 62331 A1 discloses a radiation delivery catheter that is compatible with a transfer device wherein the radiation delivery catheter is a component of a system designed to reduce the frequency of restenosis in coronary arteries after ballon angioplasty or other interventional procedures.

JP 2009 268648 A discloses a medical catheter which includes flexibility by suppressing the rigidity of a portion of an imaging marker and which includes X-ray imaging property easy to confirm.

JP 2012 196275 A discloses a catheter with a tubular catheter body having a braid wound by a plurality of stranded wires in a mesh shape, in which at least one of the stranded wires is radiopaque.

EP 0 033 659 A2 discloses a medical-surgical catheter comprising an extruded tube of flexible material including a plastic material transparent to X-ray radiation and defining the entire interior and exterior surfaces of the tube.

US 5 253 653 A discloses a guidewire assembly for measuring the size of occlusions in blood vessels which includes a guidewire having a flexible, distal end disposable in a blood vessel. The guidewire has a core wire disposed within it which extends to the distal tip.

### Summary

### Technical Problem

However, the above-described conventional catheters have a problem that the rigidity of the region where the radiopaque marker is provided is extremely higher than that of other regions.

To be more specific, the distal end portion of the conventional catheter is formed of resin having high flexibility to facilitate insertion into a body. However, when a radiopaque marker formed of metal materials such as platinum is provided at the distal end portion of the catheter, the rigidity of the portion becomes higher, causing a gap in rigidity at a border area between the portion where the radiopaque marker is provided and a portion where the radiopaque marker is not provided. Therefore, when a technician operates the conventional catheter to be curved repeatedly in the body, for example, stress is concentrated excessively at the border area between the portion where the radiopaque marker is provided and the portion where the radiopaque marker is not provided, causing a problem that the catheter is easily broken at such a border area.

Thus, the present invention aims at providing a catheter with a radiopaque marker, capable of preventing a break of the catheter by preventing excessive concentration of stress and securing the flexibility of a distal end portion of the catheter even when the catheter is inserted and curved in a blood vessel.

### Solution to Problem

In order to achieve the above-described object, the first aspect of the present invention is a catheter, including a tubular body and a coil or a braid arranged along a long axis direction of the tubular body is provided in the tubular body and a radiopaque marker arranged in the tubular body, in which , the radiopaque marker is fixed to a distal end of the coil or the braid so that a gap is provided between the radiopaque marker and the tubular body in a radial direction of the tubular body. The gap between the radiopaque marker and the tubular body may be provided on at least one of the inner side and outer side (i.e., on the inner side, on the outer side, or on the inner side and the outer side) of the radiopaque marker in the radial direction of the tubular body. Further, the gap corresponds to a hollow space formed within the tubular body, where a part of the radiopaque marker is not in contact with the tubular body.

In such a configuration, in the catheter including a tubular body and a radiopaque marker arranged in the tubular body, the radiopaque marker is fixed so that a gap is provided between the radiopaque marker and the tubular body in a radial direction of the tubular body. Thus, even when the catheter is inserted and curved in a blood vessel, it is possible to prevent excessive concentration of stress and secure the flexibility of the distal end portion of the catheter, preventing a break of the catheter. Further, in such a configuration, the reinforcing member arranged along a long axis direction of the tubular body is provided in the tubular body, and the radiopaque marker is fixed to a distal end of the reinforcing member. Thus, it is possible to prevent displacement of the radiopaque marker and securely prevent excessive concentration of stress, securely preventing a break, in addition to the effect of the first aspect or the second aspect of the present invention.

Moreover, the second aspect of the present embodiment is the catheter according to the first aspect of the invention, in which the gap is provided on both sides in the radial direction relative to the radiopaque marker.

In such a configuration, the gap is provided on both sides in the radial direction relative to the radiopaque marker. Thus, even when the catheter is inserted and curved in a blood vessel, it is possible to further prevent excessive concentration of stress and secure the flexibility of the distal end portion of the catheter, further preventing a break of the catheter, in addition to the effect of the first aspect of the present invention.

The catheter of the present invention can prevent a break of the catheter by preventing excessive concentration of stress and securing the flexibility of a distal end portion of the catheter even when the catheter is inserted and curved in a blood vessel.

### Brief Description of Drawings

FIG. 1 is a plan view of a catheter according to a first embodiment of the present invention.
FIG. 2 is a partial enlarged section of the catheter according to the first embodiment.
FIG. 3 is a partial enlarged section illustrating a modification of the catheter according to the first embodiment.
FIG. 4 is a partial enlarged section of a catheter according to a second embodiment.
FIG. 5 is a partial enlarged section of a catheter according to a third embodiment.
FIG. 6 is a partial enlarged section of an exemplary catheter.

### Description of Embodiments

### [First embodiment]

In the following, the catheter according to embodiments of the present invention will be described with reference to the enclosed drawings.

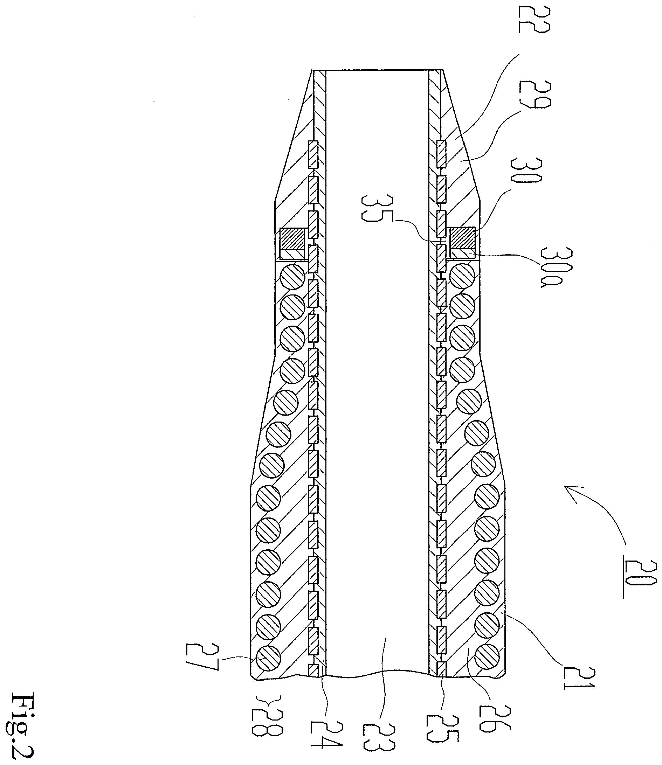

FIG. 1 is a plan view of a catheter according to the first embodiment of the present invention, and FIG. 2 is a partial enlarged section of the catheter according to the first embodiment.

As illustrated in FIG. 1, a catheter 1 includes an operation part 10 operated by a technician and a catheter tube (corresponding to a "tubular body" of the present invention) 20 connected to a distal end of the operation part 10. Moreover, the catheter tube 20 includes a catheter main body part 21 extended from the operation part 10 and a distal end tip 22 positioned on the distal end side of the catheter main body part 21. Each of the vicinity of the distal end of the catheter main body part 21 and the vicinity of the distal end of the distal end tip 22 has a tapered form in which the outer diameter becomes smaller toward the distal end.

Moreover, as illustrated in FIG. 2, the catheter main body part 21 has a multilayer structure, and includes an inner layer 24, a braid 25, and an outer layer 26 in the order from the inner side in a radial direction relative to a center axis of the catheter main body part 21.

The inner layer 24 has a tubular form with a lumen 23, and is arranged over the catheter main body 21 and the distal end tip 22. Note that the inner layer 24 is preferably formed of fluororesin such as polytetrafluoroethylene having small sliding resistance. Moreover, the inner layer 24 may have a multilayer structure partially or entirely.

Moreover, the braid 25 is arranged at the periphery of the inner layer 24. The distal end of the braid 25 is arranged in the distal end tip 22, and positioned on the proximal end side than the distal end of the inner layer 24. Although the braid 25 formed of a plurality of pieces of wire is arranged at the periphery of the inner layer 24 in the present embodiment, a coil wound by a single piece of wire or a plurality of pieces of wire may be arranged. In each case, the wire may be a single strand wire (i.e., be formed of a single strand) or a multiple strand wire (i.e., be formed of multiple strands). The braid 25 is formed of materials such as stainless steel, superelastic alloy such as an Ni-Ti alloy, piano wire, or tungsten wire.

Moreover, the outer layer 26 is arranged to cover the periphery of the inner layer 24 and the braid 25, and the distal end of the outer layer 26 forms the distal end of the catheter main body part 21. In the outer layer 26, a reinforcing member 27 formed of a coil is arranged coaxially with the outer layer 26. Then, the reinforcing member 27 and the outer layer 26 form a reinforcing layer 28. Note that the distal end of the reinforcing member 27 extends to the distal end of the catheter main body part 21. In the present embodiment, a coil is used as the reinforcing member 27. However, a braid formed by braiding a plurality of pieces of wire may be used. Also in this case, the wire may be a single strand wire (i.e., be formed of a single strand) or a multiple strand wire (i.e., be formed of multiple strands). The reinforcing member 27 is formed of materials such as stainless steel, superelastic alloy such as an Ni-Ti alloy, piano wire, or tungsten wire.

The resin materials forming the outer layer 26 include polyamide, polyamide elastomer, polyester, polyurethane, or the like, for example. In the present embodiment, polyurethane is used. Moreover, the outer layer 26 may have a multilayer structure partially or entirely.

The distal end tip 22 has a multilayer structure, and includes, from the inner side in a radial direction relative to a center axis line of the distal end tip 22, the inner layer 24, the braid 25, and a distal end outer layer 29 formed continuously from the outer layer 26. The resin materials forming the distal end outer layer 29 include polyamide, polyamide elastomer, polyester, polyurethane, or the like, for example, similarly to the outer layer 26. Note that polyurethane is used for the distal end outer layer 29 of the present embodiment. When the main part of the distal end tip 22 is formed of polyurethane in this manner, damages to blood vessel inner walls can be prevented. In order to improve the visibility of the distal end tip 22, the distal end outer layer 29 preferably contains radiopaque tungsten powder, and the like.

Moreover, a ring-formed radiopaque marker 30 formed of a platinum material is arranged in the distal end tip 22, and the proximal end of the radiopaque marker 30 is fixed to the distal end of the reinforcing member 27 through a fused part 30a. Concretely, the proximal end of the radiopaque marker 30 and the distal end of the reinforcing member 27 are fused with each other by YAG welding. In the present embodiment, platinum is used for the radiopaque marker 30. However, other radiopaque metal materials may be used. Moreover, in the present embodiment, the proximal end of the radiopaque marker 30 and the distal end of the reinforcing member 27 are fused with each other. However, they may be fixed to each other by other fixing methods such as an adhesive.

Moreover, as illustrated in FIG. 2, a gap 35 is provided between the inner layer 24 and the radiopaque marker 30 on the inner side in a radial direction of the catheter tube 20, and a part of the outer peripheral surface of the radiopaque marker 30 is exposed to the gap 35. That is, a surface on the side of the inner layer 24 of the outer peripheral surface of the radiopaque marker 30 is not in contact with other members.

In the catheter 1, the radiopaque marker 30 is fixed so that the gap 35 is provided between the radiopaque marker 30 and the catheter tube 20 on the inner side in a radial direction of the catheter tube 20. Thus, even when the catheter 1 is inserted and curved in a blood vessel, the gap 35 can sufficiently absorb deformation of the inner layer 24, the distal end outer layer 29, and the like around the radiopaque marker 30, and prevent excessive concentration of stress. In this manner, it is possible to secure the flexibility of the distal end portion of the catheter 1 and prevent a break of the catheter 1.

Furthermore, as described above, the radiopaque marker 30 is fused with the distal end of the reinforcing member 27. Thus, it is possible to prevent displacement of the radiopaque marker 30 and securely prevent excessive concentration of stress, thus securely preventing a break of the catheter 1. Moreover, also in the process for manufacturing the catheter tube 20, the distal end outer layer 29 can be formed in the state where the radiopaque marker 30 is fused with the distal end of the reinforcing member 27. Thus, it is possible to prevent displacement of the radiopaque marker 30 and produce the catheter 1 with high dimensional accuracy.

Furthermore, the reinforcing member 27 and the radiopaque marker 30 are fused with each other, whereby the resin material forming the distal end outer layer 29 hardly enters the gap 35 formed on the side of the inner layer 24 of the radiopaque marker 30. Thus, it is possible to form the gap 35 easily.

Moreover, the gap 35 is also formed between the fused part 30a (distal end of the reinforcing member 27) and the inner layer 24. Thus, when the catheter 1 is inserted and curved in a blood vessel, it is possible to further prevent excessive concentration of stress and secure the flexibility of the distal end portion of the catheter 1, further preventing a break of the catheter 1.

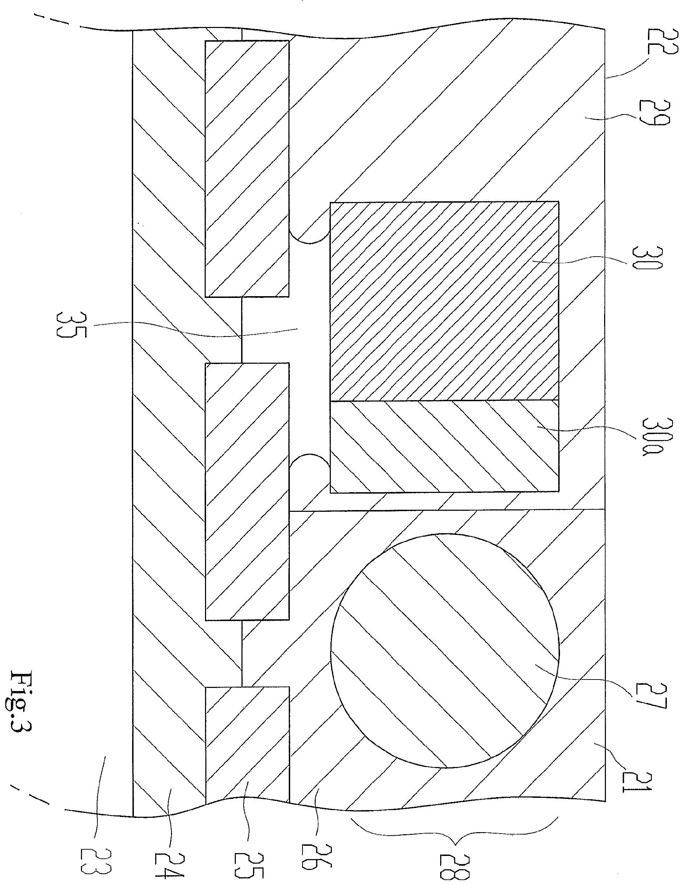

FIG. 3 is a partial enlarged section illustrating a modification of the catheter according to the first embodiment. In FIG. 3, the distal end outer layer 29 partially enters the inside of the gap 35 provided between the radiopaque marker 30 and the inner layer 24. In such a configuration, the radiopaque marker 30 is fused with the reinforcing member 27 through the fused part 30a and, in addition, supported from the lower side by the distal end outer layer 29. Thus, the displacement of the radiopaque marker 30 is further prevented.

The present invention having such a configuration is a catheter including a tubular body and a radiopaque marker arranged in the tubular body, in which the radiopaque marker is fixed in a radial direction of the tubular body so that a gap is provided between the radiopaque marker and the tubular body and so that the tubular body enters an end edge portion of the radiopaque marker.

### [Second embodiment]

In the following, the catheter according to the second embodiment will be described. The parts common to the catheter according to the first embodiment will be represented with same symbols in the drawings, and the explanation thereof will be simplified or omitted.

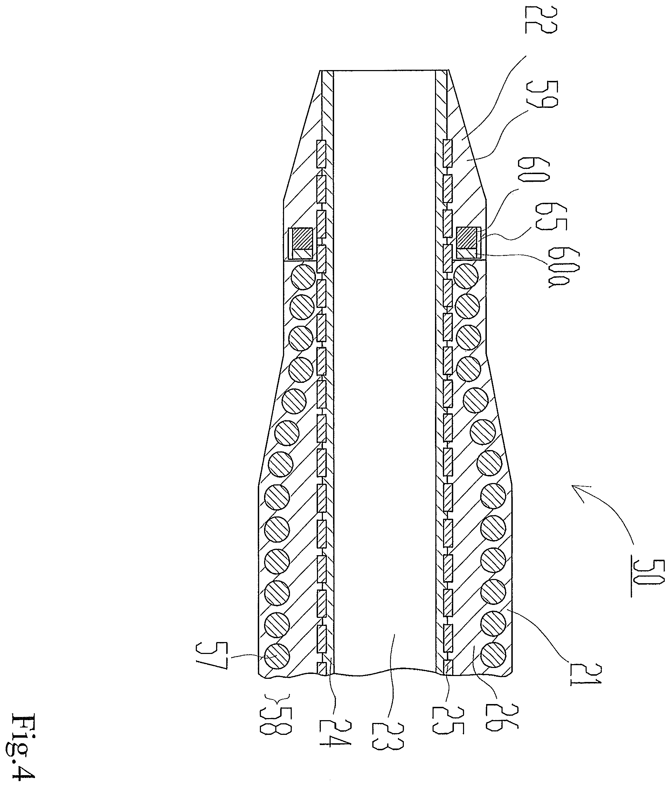

FIG. 4 is a partial enlarged section of the catheter according to the second embodiment. In FIG. 4, a catheter tube 50 is different from the catheter tube 20 of the first embodiment in the positional relation between the radiopaque marker and the gap.

That is, although the proximal end of a radiopaque marker 60 arranged on the distal end tip 22 is fused with the distal end of a reinforcing member 57 forming a reinforcing layer 58 through a fused part 60a, the gap 35 of the catheter tube 20 of the first embodiment is arranged on the inner side in a radial direction of the catheter tube 20 relative to the radiopaque marker 30, while a gap 65 of the present embodiment is arranged on the outer side in a radial direction of the catheter tube 50 relative to the radiopaque marker 60 (between the radiopaque marker 60 and a distal end outer layer 59).

In such a configuration, when the catheter is inserted and curved in a blood vessel, it is possible to prevent excessive concentration of stress and secure the flexibility of the distal end portion of the catheter, preventing a break of the catheter. Moreover, the gap 65 is also formed between the fused part 60a (distal end of the reinforcing member 57) and the distal end outer layer 59. Thus, when the catheter is inserted and curved in a blood vessel, it is possible to further prevent excessive concentration of stress and secure the flexibility of the distal end portion of the catheter, further preventing a break of the catheter.

### [Third embodiment]

In the following, the catheter according to the third embodiment will be described. The parts common to the catheter according to the first embodiment and the catheter according to the second embodiment will be represented with same symbols in the drawings, and the explanation thereof will be simplified or omitted.

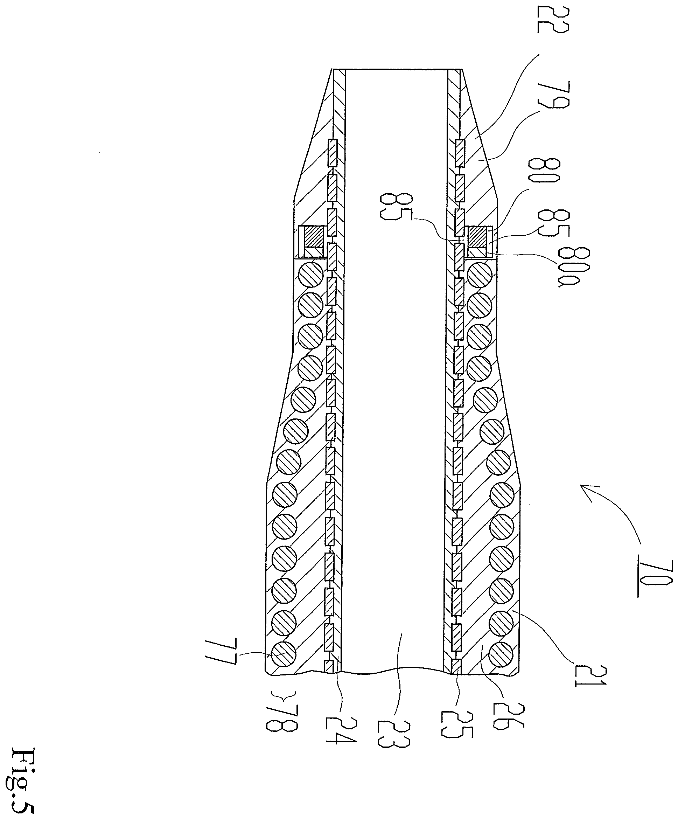

FIG. 5 is a partial enlarged section of the catheter according to the third embodiment. In FIG. 5, a catheter tube 70 is different from the catheter tube 20 of the first embodiment in the positional relation between the radiopaque marker and the gap.

That is, although the proximal end of a radiopaque marker 80 arranged at the distal end tip 22 is fused with the distal end of a reinforcing member 77 forming a reinforcing layer 78 through a fused part 80a, a gap 85 of the present embodiment is arranged on the both sides in a radial direction of the catheter tube 70 relative to the radiopaque marker 80 (between the radiopaque marker 80 and the inner layer 24 and between the radiopaque marker 80 and a distal end outer layer 79).

In such a configuration, when the catheter is inserted and curved in a blood vessel, it is possible to further prevent excessive concentration of stress and secure the flexibility of the distal end portion of the catheter, further preventing a break of the catheter.

### [exemplary catheter]

In the following, the catheter according to an exemplary catheter will be described. The parts common to the catheter according to the first embodiment, the catheter according to the second embodiment, and the catheter according to the third embodiment will be represented with same symbols in the drawings, and the explanation thereof will be simplified or omitted.

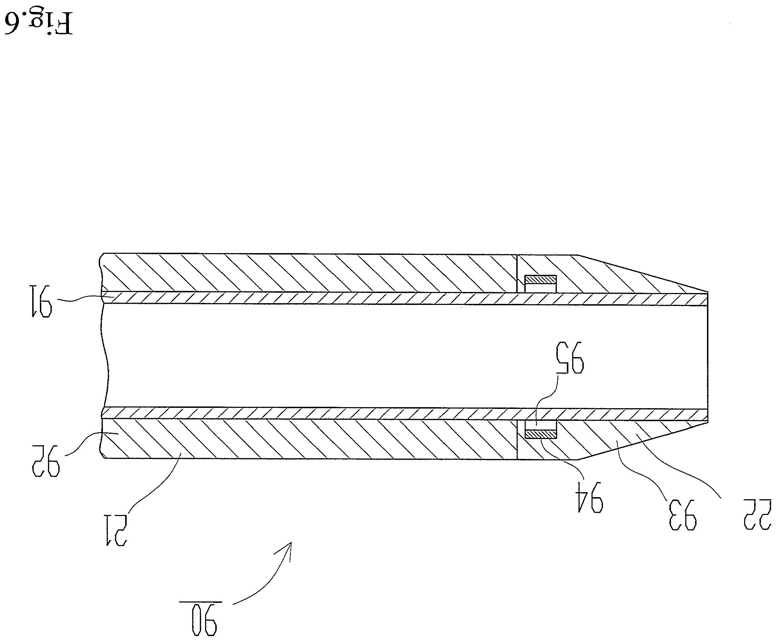

FIG. 6 is a partial enlarged section of an exemplary catheter. In FIG. 6, a catheter tube 90 includes the catheter main body part 21 and the distal end tip 22. The catheter main body part 21 includes an inner layer 91 and an outer layer 92 covering the periphery of the inner layer 91, and the distal end chip 22 includes an inner layer 91 extended from the catheter main body part 21 and a distal end outer layer 93 covering the periphery of the inner layer 91.

A radiopaque marker 94 arranged at the distal end tip 22 is arranged between the inner layer 91 and the distal end outer layer 93, and a gap 95 is provided between the radiopaque marker 94 and the inner layer 91.

In such a configuration, when the catheter is inserted and curved in a blood vessel, it is possible, with the gap 95 on the inner side of the radiopaque marker 94, to prevent excessive concentration of stress and secure the flexibility of the distal end portion of the catheter, preventing a break of the catheter.

In the present example, the gap 95 is provided between the radiopaque marker 94 and the inner layer 91. However, similarly to the catheters according to the second embodiment and the third embodiment, the gap 95 may be provided on the outer side in a radial direction of the catheter tube 90 relative to the radiopaque marker 94 (between the radiopaque marker 94 and the distal end outer layer 93) or on the both sides in a radial direction of the catheter tube 90 relative to the radiopaque marker 94 (between the radiopaque marker 94 and the inner layer 91 and between the radiopaque marker 94 and the distal end outer layer 93). Also in such cases, the same effect as the effect described in each embodiment is exerted.

In the first configuration of the exemplary catheter, the configuration in which the radiopaque marker is arranged on the side of the distal end tip has been described. However, the radiopaque marker may be arranged on the side of the catheter tube, and also in such a case, the same effect as the effect described in each example is exerted.

Moreover, in the first configuration of the exemplary catheter, the catheter is divided to the catheter tube and the distal end tip. However, the catheter may be configured so that the catheter tube and the distal end tip are formed integrally with each other, and also in such a case, the same effect as the effect described in each example is exerted.

Furthermore, in the first configuration of the exemplary catheter, the shape of the radiopaque marker is a ring shape. However, the shape may be other shapes such as a coil shape or a ring shape with a partial cutout. However, with a ring shape, even when the distal end of the catheter is curved in any direction, it is possible to prevent excessive concentration of stress and secure the flexibility of the distal end portion of the catheter, preventing a break of the catheter.

### Reference Signs List

- 1: catheter
- 10: operation part
- 20, 50, 70, 90: catheter tube
- 21: catheter main body part
- 22: distal end tip
- 24, 91: inner layer
- 27, 57, 77: reinforcing member
- 28, 58, 78: reinforcing part
- 29, 59, 79, 93: distal end outer layer
- 30, 60, 80, 94: radiopaque marker
- 30a, 60a, 80a: fused part
- 35, 65, 85, 95: gap
- 92: outer layer

## Claims

1. A catheter (1), comprising:
a tubular body (20, 50, 70),a radiopaque marker (30, 60, 80) arranged in the tubular body (20, 50, 70);and
a coil or a braid (27, 57, 77) arranged along a long axis direction of the tubular body (20, 50, 70) in the tubular body (20, 50, 70), **characterized in that**
the radiopaque marker (30, 60, 80) is fixed to a distal end (30a) of the coil or braid (27, 57, 77) so that a gap (35, 65, 85) is provided between the radiopaque marker (30, 60, 80) and the tubular body (20, 50, 70) in a radial direction of the tubular body (20, 50, 70).

2. The catheter according to claim 1, wherein the gap (85) is provided on both sides in the radial direction relative to the radiopaque marker (80).

## Patentansprüche

1. Katheter (1) mit:
einem röhrenförmigen Körper (20, 50, 70),
einem radiopaken Marker (30, 60, 80), der in dem röhrenförmigen Körper (20, 50, 70) angeordnet ist; und
einer Wicklung oder einem Geflecht (27, 57, 77), die oder das in dem röhrenförmigen Körper (20, 50, 70) in Längsrichtung desselben angeordnet ist, **dadurch gekennzeichnet, dass**
der radiopake Marker (30, 60, 80) an einem distalen Ende (30a) der Wicklung oder des Geflechts (27, 57, 77) dergestalt befestigt ist, dass zwischen dem radiopaken Marker (30, 60, 80) und dem röhrenförmigen Körper (20, 50, 70) eine Lücke (35, 65, 85) in radialer Richtung des röhrenförmigen Körpers (20, 50, 70) vorgesehen ist.

2. Katheter nach Anspruch 1, wobei die Lücke (85) in der radialen Richtung beiderseits des radiopaken Markers (80) vorgesehen ist.

## Revendications

1. Cathéter (1), comprenant :
un corps tubulaire (20, 50, 70), un marqueur radio-opaque (30, 60, 80) disposé dans le corps tubulaire (20, 50, 70) ; et
une bobine ou une tresse (27, 57, 77) disposée le long d'une direction de grand axe du corps tubulaire (20, 50, 70) dans le corps tubulaire (20, 50, 70), **caractérisé en ce que**
le marqueur radio-opaque (30, 60, 80) est fixé à une extrémité distale (30a) de la bobine ou tresse (27, 57, 77) de sorte qu'un interstice (35, 65, 85) est formé entre le marqueur radio-opaque (30, 60, 80) et le corps tubulaire (20, 50, 70) dans une direction radiale du corps tubulaire (20, 50, 70).

2. Cathéter selon la revendication 1, où l'interstice (85) est formé des deux côtés dans la direction radiale par rapport au marqueur radio-opaque (80).
